# EUROPEAN PATENT APPLICATION

(11) **EP 4 582 746 A1**
(43) Date of publication of application: **09.07.2025**
(21) Application number: 22957121.1
(22) Date of filing: 27.10.2022
(51) Int. Cl.: F24F 1/0076, F24F 8/20, F24F 8/22, F24F 1/0063, F24F 13/32

(54) **AIR CONDITIONER**

(30) Priority: 31.08.2022 CN 202211056723; 31.08.2022 CN 202222314458 U
(71) Applicant: GD Midea Air-Conditioning Equipment Co., Ltd., Foshan, Guangdong 528311 (CN)
(72) Inventor: ZHAO, Peng, Foshan, Guangdong 528311 (CN); ZHANG, Fan, Foshan, Guangdong 528311 (CN); LI, Baohua, Foshan, Guangdong 528311 (CN); ZHANG, Weidong, Foshan, Guangdong 528311 (CN)
(74) Representative: RGTH
(86) International application number: PCT/CN2022/128054
(87) International publication number: WO 2024/045305

(57) **Abstract**

Provided is an air conditioner (100). The air conditioner (100) includes a casing (1), an indoor heat exchanger (2), an indoor fan (3), and a sterilization module (4). The casing (1) defines an indoor air duct (100a) therein. The indoor heat exchanger (2) and the indoor fan (3) are disposed in the indoor air duct (100a). The sterilization module (4) is disposed in the casing (1) and includes a pulse unit (41). The pulse unit (41) is configured to output pulsed intense light to at least sterilize an airflow in the indoor air duct (100a).

## Description

### CROSS-REFERENCE TO RELATED APPLICATION

The present application claims priorities to Chinese patent applications Nos. 202211056723.4 and 202222314458.7, both titled "AIR CONDITIONER" and filed by GD MIDEA AIR-CONDITIONING EQUIPMENT CO., LTD. on August 31, 2022.

### FIELD

The present disclosure relates to the field of air conditioner technologies, and more particularly, to an air conditioner.

### BACKGROUND

In the related art, air conditioners mostly use ultraviolet lamp devices for sterilization, but the penetrability of ultraviolet light is not strong, and it is impossible to achieve comprehensive and effective sterilization of the air conditioner.

### SUMMARY

The present disclosure aims to solve at least one of the technical problems in the related art to some extent.

To this end, the present disclosure provides an air conditioner. The air conditioner performs sterilization treatment on air entering an indoor air duct, in such a manner that indoor air maintains ultra-high cleanliness, which meets a user's demand for clean air, and enhances use experience.

According to an embodiment of the present disclosure, an air conditioner comprises: a casing defining an indoor air duct therein; an indoor heat exchanger disposed in the indoor air duct; an indoor fan disposed in the indoor air duct; and a sterilization module disposed in the casing, the sterilization module comprising a pulse unit configured to output pulsed intense light to at least sterilize an airflow in the indoor air duct.

With the air conditioner according to the embodiment of the present disclosure, the sterilization module performs sterilization treatment on the airflow entering the indoor air duct, in such a manner that indoor air maintains the ultra-high cleanliness, which meets the user's demand for the clean air, and enhances the use experience. In addition, sterilization is achieved with low energy consumption.

### BRIEF DESCRIPTION OF THE DRAWINGS

FIG. 1 is a schematic view of an air conditioner according to an embodiment of the present disclosure.
FIG. 2 is a sectional view taken along line A-A in FIG. 1.
FIG. 3 is another schematic view of the air conditioner illustrated in FIG. 1.
FIG. 4 is yet another schematic view of the air conditioner illustrated in FIG. 1.
FIG. 5 is a sectional view taken along line B-B in FIG. 4.
FIG. 6 is another schematic view of the air conditioner illustrated in FIG. 4.
FIG. 7 is yet another schematic view of the air conditioner illustrated in FIG. 4.
FIG. 8 is a sectional view taken along line C-C in FIG. 5.
FIG. 9 is a schematic view of a pulse unit illustrated in FIG. 1.
FIG. 10 is a main view of the pulse unit illustrated in FIG. 9.
FIG. 11 is a side view of the pulse unit illustrated in FIG. 9.
FIG. 12 is a top view of the pulse unit illustrated in FIG. 9.
FIG. 13 is a sectional view taken along line D-D in FIG. 12.
FIG. 14 is a schematic view of a pulse light source illustrated in FIG. 9.
FIG. 15 is a schematic view of a circuit unit illustrated in FIG. 1.
FIG. 16 is a sectional view taken along line E-E in FIG. 15.
FIG. 17 is a side view of the circuit unit illustrated in FIG. 15.
FIG. 18 is another side view of the circuit unit illustrated in FIG. 15.

### DETAILED DESCRIPTION OF THE EMBODIMENTS

Embodiments of the present disclosure will be described in detail below with reference to examples thereof as illustrated in the accompanying drawings, throughout which same or similar elements, or elements having same or similar functions, are denoted by same or similar reference numerals. The embodiments described below with reference to the drawings are illustrative only, and are intended to explain, rather than limit, the present disclosure.

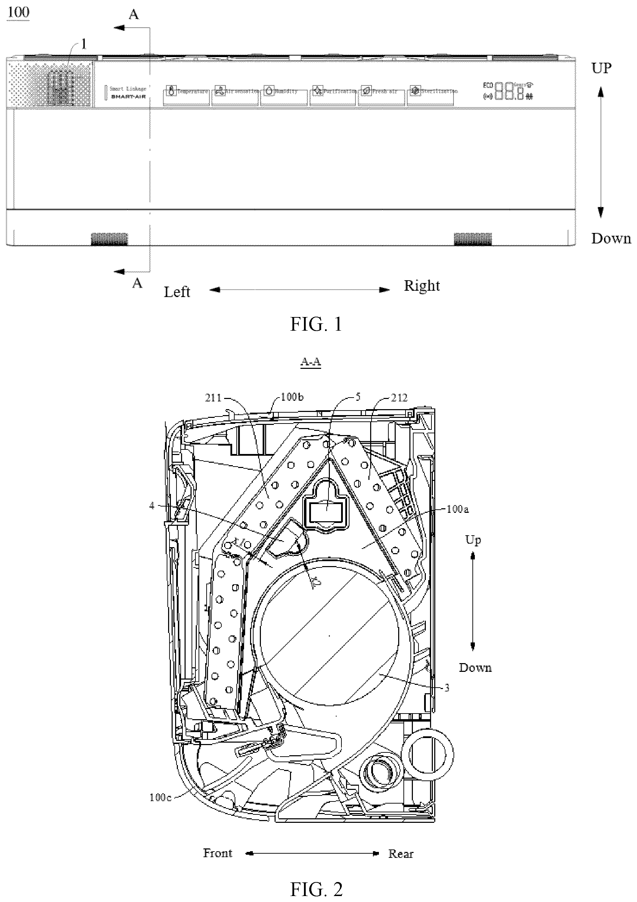

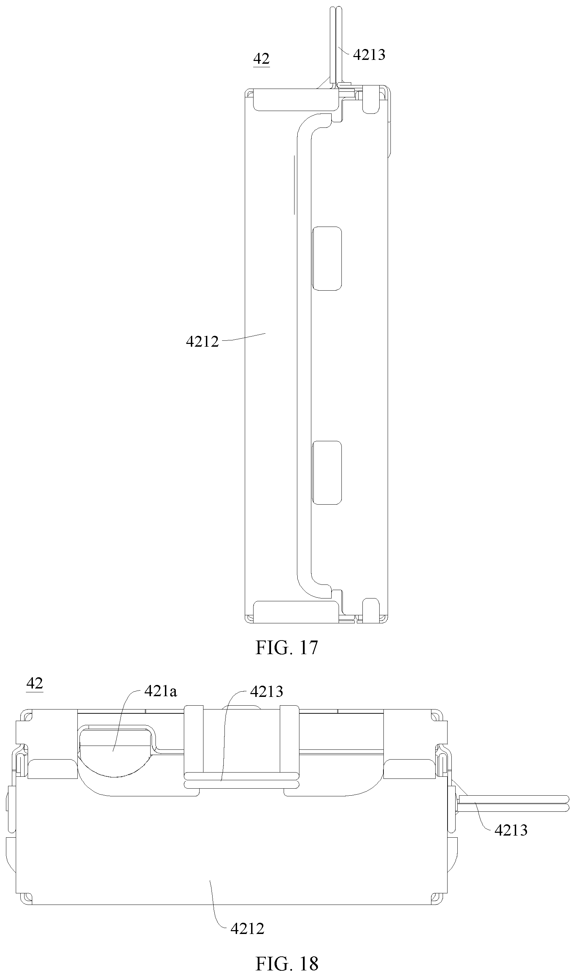

An air conditioner 100 according to the embodiments of the present disclosure is described below with reference to FIG. 1 to FIG. 18.

As illustrated in FIG. 1 to FIG. 3, the air conditioner 100 comprises a casing 1, an indoor heat exchanger 2, an indoor fan 3, and a sterilization module 4. The casing 1 defines an indoor air duct 100a therein. The indoor air duct 100a has an indoor air inlet 100b and an indoor air outlet 100c. The indoor heat exchanger 2 and the indoor fan 3 are disposed in the indoor air duct 100a. Under a driving of the indoor fan 3, indoor air can flow through the indoor air inlet 100b to the indoor air duct 100a, exchange heat with the indoor heat exchanger 2, and finally flow into an indoor space through the indoor air outlet 100c, which realizes circulation of the indoor air and at least allows for an adjustment of an indoor temperature.

The sterilization module 4 is disposed in the casing 1 and can be directly or indirectly connected to the casing 1. The sterilization module 4 is configured to at least sterilize an airflow in the indoor air duct 100a (e.g., cold air or hot air in the indoor air duct 1a) to improve a quality of the indoor air.

Therefore, the air conditioner 100 has a sterilization function to ensure the quality of the air flowing into the indoor space, enriching functionality of the air conditioner 100.

The sterilization module 4 comprises a pulse unit 41 configured to output pulsed intense light to at least sterilize the airflow in the indoor air duct 100a. The pulsed intense light is used to irradiate the airflow in the indoor air duct 100a to achieve highly efficient sterilization of the indoor air duct 100a. The pulsed intense light can remove gaseous pollutants (such as formaldehyde, toluene, or acetic acid) in the indoor air duct 100a, in such a manner that the indoor air maintains ultra- high cleanliness, which effectively improves the quality of the indoor air, meets a user's demand for clean air, and enhances use experience.

Therefore, with the pulsed intense light outputted by the pulse unit 41, great damages are caused to microorganisms such as bacteria, which reduces internal enzyme activity or even destroy DNA and RNA structures of the microorganisms, leading to death of the microorganisms such as bacteria. In addition, white intense light is outputted by the pulse unit 41 in a form of pulses to at least irradiate air in the indoor air duct 100a, for killing the bacteria. In addition, during sterilization process, there is no need to heat the air, which reduces energy consumption of the sterilization module 4 and therefore lowers cost of use for the user.

The pulsed intense light comprises multi-spectrum light, comprising ultraviolet light, infrared light, and visible light. The ultraviolet light has an important role in sterilization, while the infrared light provides synergistic sterilization, in such a manner that the sterilization module 4 has a satisfactory sterilization effect and a satisfactory sterilization efficiency, which facilitates ensuring a sterilization ability of the sterilization module 4, and further reduces the energy consumption of the sterilization module 4. The ultraviolet light in the pulsed intense light can destroy genetic materials of the microorganisms such as bacteria, leading to death of the bacteria. The infrared light in the pulsed intense light can rapidly increase a temperature of the bacteria, leading to dehydration and death of the bacteria. The visible light in the pulsed intense light can destroy protein structures of the bacteria through a pulse flash effect, high penetrability, and high impact of the visible light, which causes protein denaturation and leads to death of the bacteria, offering a satisfactory sterilization efficiency and satisfactory compatibility.

Therefore, the pulsed intense light outputted by the sterilization module 4 can at least realize safe (mercury-free), effective, and energy-efficient cold sterilization (i.e., non-heating sterilization) for the airflow in the indoor air duct 100a. During the sterilization for the airflow, there is no need to heat the airflow, which is conducive to reducing the energy consumption of the sterilization, and also has a substantially minimal impact on a temperature and other characteristics of the airflow, thereby facilitating the air conditioner 100 to adjust the indoor temperature or the like. In addition, when the air conditioner 100 is used for cooling, a substantially negligible effect of the sterilization module 4 on the temperature of the airflow facilitates maintenance of a cooling efficiency of the air conditioner 100 and reduces the energy consumption of the air conditioner 100 associated with cooling. In addition, the sterilization module 4 produces small environmental pollution when in operation, which facilitates provision of a friendly use environment for the user, and poses no harm to humans, helping to ensure health of the user.

With the air conditioner 100 according to the embodiments of the present disclosure, the pulse unit 41 outputs the pulsed intense light to realize the sterilization. Due to the high penetrability of the pulsed intense light, the sterilization efficiency and the sterilization effect for the indoor air duct 100a can be improved to realize highly efficient sterilization of the indoor air duct 100a, making the indoor air cleaner, and improving the quality of the indoor air. Compared with the ultraviolet ray sterilization technology in the related art, the sterilization module 4 in the present disclosure can sterilize a wider variety of microorganisms, saving sterilization time, and realizing low energy consumption of the sterilization module 4.

In the related art, the ultraviolet ray sterilization relies on a photochemical effect, which constitutes a main sterilization mechanism of the ultraviolet ray sterilization. The photochemical effect refers to an optical reaction produced when microorganisms receive light irradiation of a specific wavelength. When double-stranded genetic information in the microorganisms such as bacteria is irradiated with ultraviolet light, pyrimidine and isomers of the pyrimidine are formed. These substances disrupt metabolic functions of the microorganisms and cause genetic abnormalities of the microorganisms, eventually leading to the death of the microorganisms.

The pulsed intense light employs a photothermal sterilization mechanism. A photothermal effect refers to a temperature increase of a substance after the substance absorbs light energy. When exposed to the pulsed intense light at close range, the microorganisms rapidly absorb a large amount of light energy in a short period, and thus a surface temperature of the microorganisms increases rapidly, which results in complete destruction of a surface structure of the microorganisms, leading to death of the microorganisms. Since an entire photothermal process is extremely short, an object being irradiated does not experience a temperature increase. Therefore, the pulsed intense light, which employs the photothermal sterilization mechanism, can effectively kill all types of microorganisms.

The table below reveals a comparison between pulsed intense light sterilization and ultraviolet light sterilization. Since the pulsed intense light encompasses an ultraviolet wavelength spectrum, the pulsed intense light also possesses a photochemical sterilization mechanism. However, in the pulsed intense light, the photochemical sterilization mechanism plays only a supplementary role. For microorganisms such as molds and spores, ultraviolet rays struggle to penetrate dense cell wall structures, and thus DNA cannot absorb the ultraviolet rays. Consequently, the ultraviolet ray sterilization is much less efficient than the pulsed intense light sterilization.

| Project | Direct current ultraviolet ray sterilization | Pulsed intense light sterilization |
|---|---|---|
| Main sterilization mechanism | Photochemical sterilization | Photothermal sterilization |
| Environmental protection | Containing mercury | Mercury-free and no toxic by-products |
| Spectral sterilization | Many ultraviolet "immune" bacteria exist | Broad-spectrum effectiveness |
| e | Typically up to 99.99% | Single pulse>99.9999% |
| Photoreactivation of lethal microorganisms | Yes | No |
| Adjustable energy output | No | Yes |
| Single lamp power density | Generally 0.02 kw to 0.1 kw | 0.5 kw to 10 kw, widely adjustable |
| Light degradation | Severe | Controllable through a real-time parameter adjustment |
| Preheating required | Yes | No |

The pulse unit 41 ionizes inert gases through a high-voltage power supply, exciting photons to produce instantaneous high-intensity light. Typically, the pulsed intense light comprises multi-spectrum light ranging from 100 nm to 1 mm. The pulse unit 41 comprises a xenon lamp 4121. The pulsed intense light is generated by applying a high voltage to the xenon lamp 4121, causing rapid ionization of a xenon gas inside the xenon lamp 4121 within an extremely short time period. In this way, the xenon gas releases the instantaneous high-intensity light produced through exciting the photons in a form of high-intensity light radiation. The pulse unit 41 is not limited in this regard.

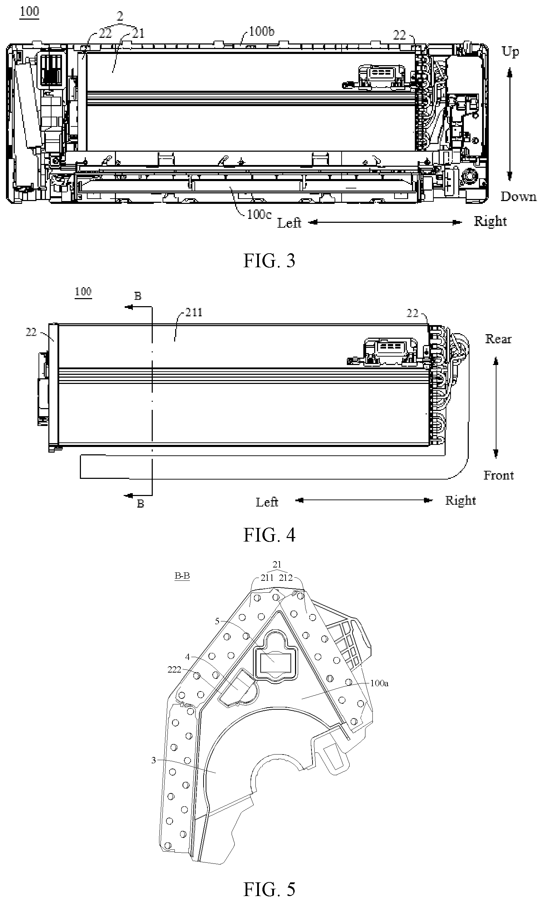

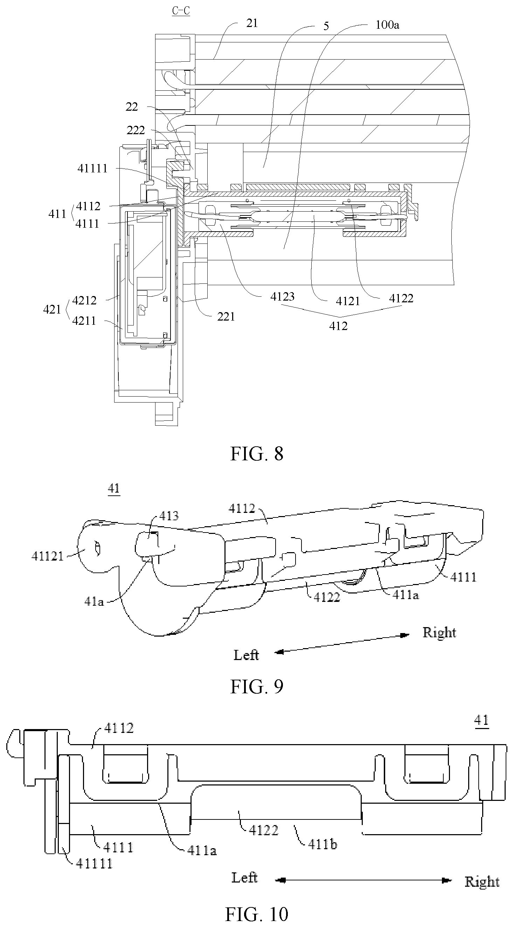

In some embodiments of the present disclosure, as illustrated in FIG. 3 and FIG. 8, the indoor heat exchanger 2 comprises a heat exchanger body 21 and two support members 22. The two support members 22 are respectively disposed at two ends of the heat exchanger body 21 in a length direction of the heat exchanger body 21. At least one of the two support members 22 is provided with the sterilization module 4. In this way, stability of the sterilization module 4 at the air conditioner 100 can be improved, which therefore ensures operation stability of the sterilization module 4, and facilitates a centralized arrangement of the indoor heat exchanger 2 and the sterilization module 4. In addition, the sterilization module 4 is disposed in at least one of two ends of the indoor heat exchanger 2 in a length direction of the indoor heat exchanger 2, in such a manner that the indoor heat exchanger 2 and the sterilization module 4 have a compact structure, which is conducive to saving a space occupied by the indoor heat exchanger 2 and the sterilization module 4, improving a utilization rate of a space in the air conditioner 100.

At least part of the pulse unit 41 is located at a side of a corresponding support member 22 of the two support members 22 facing towards the other support member 22 of the two support members 22. For example, a part or all of the pulse unit 41 is located at the side of the corresponding support member 22 facing towards the other support member 22, which facilitates an arrangement of the pulse unit 41 in a space adjacent to the indoor heat exchanger 2, and is conducive to expanding irradiation coverage of the pulsed intense light outputted by the pulse unit 41 in the indoor air duct 100a. In this way, an effective sterilization region of the pulse unit 41 in the indoor air duct 100a is increased, ensuring a sterilization effect of the sterilization module 4.

In addition, the above arrangement of the sterilization module 4 facilitates a reasonable adjustment of the sterilization module 4 to better adapt to the air conditioner 100, e.g., a reasonable adjustment of an increase in a length of the sterilization module 4, which facilitates ensuring that the sterilization module 4 has a suitable sterilization range, and is conducive to realizing effective sterilization in the indoor air duct 100a through mounting only one sterilization module 4. In this way, manufacturing costs of the air conditioner 100 are saved, which facilitates adaptation of the sterilization module 4 to differentiated needs for sterilization of different air conditioners 100.

In examples of FIG. 3 and FIG. 8, the length direction of the heat exchanger body 21 is a left-right direction. The support member 22 at a left side is provided with the sterilization module 4. The support member 22 at a right side is provided with no sterilization module 4. Also, each of the support member 22 at the left side and the support member 22 at the right side may be provided with the sterilization module 4. Alternatively, the support member 22 at the left side is provided with no sterilization module 4, and the support member 22 at the right side is provided with the sterilization module 4.

For example, as illustrated in FIG. 8, the pulse unit 41 is arranged in an extension direction of the indoor heat exchanger 2 and extends towards the other support member 22, in such a manner that a length direction of the pulse unit 41 is consistent with the length direction of the indoor heat exchanger 2, which is conducive to increasing an irradiation area of the pulse unit 41 on the airflow in the indoor air duct 100a, further improving the sterilization effect. In addition, a reduction of a space occupied by the pulse unit 41 in a plane perpendicular to the length direction of the indoor heat exchanger 2 is facilitated, which further facilitates the arrangement of the sterilization module 4 in the air-conditioner 100.

Of course, the pulse unit 41 is not limited to being arranged in the above direction. For example, the pulse unit 41 may be arranged in a direction perpendicular to the length direction of the indoor heat exchanger 2.

In some embodiments of the present disclosure, as illustrated in FIG. 8, the support member 22 has a mounting hole 221 formed therein. The mounting hole 221 may penetrate the support member 22 in the length direction of the heat exchanger body 21. The pulse unit 41 passes through the mounting hole 221 and is fixedly connected to the support member 22. In this way, the pulse unit 41 and the support member 22 are connected as a whole. The mounting hole 221 can provide guidance and a positional restriction for the pulse unit 41 to facilitate an assembly of the pulse unit 41 and the support member 22, which is conducive to improving overall structural stability of the pulse unit 41 and the support member 22 and an assembly efficiency of the pulse unit 41 and the support member 22.

In addition, when a cable of the pulse unit 41 needs to be routed from a side of the support member 22 to another side of the support member 22, the cable of the pulse unit 41 may be extended from the side of the support member 22 to the other side of the support member 22 through the mounting hole 221. In this way, the mounting hole 221 provides constraint and guidance for the cable of the pulse unit 41, which eliminates a need for an additional cable routing hole at the support member 22, facilitating simplifying a structure and manufacturing procedures of the support member 22.

Of course, a method for an engagement between the support member 22 and the pulse unit 41 is not limited in this regard. For example, the support member 22 may not have a mounting hole 221 formed therein, and an end of the pulse unit 41 in the length direction of the pulse unit 41 is fixedly connected to the support member 22 by a fastener.

In some embodiments of the present disclosure, as illustrated in FIG. 8 and FIG. 9, the pulse unit 41 comprises a mounting bracket 411 and a pulse light source 412 disposed at the mounting bracket 411. The mounting bracket 411 is provided with an abutment portion 41111 at an end of the mounting bracket 411 in a length direction of the mounting bracket 411. The abutment portion 41111 is abutted against a side of the mounting hole 221 facing away from the other support member 22, to restrict the pulse unit 41 from moving towards the other support member 22, which facilitates a realization of a positional restriction on the pulse unit 41 and mounting of the pulse unit 41. In addition, positioning most of the pulse unit 41 at a side of the corresponding support member 22 facing towards the other support member 22 is facilitated, which facilitates the entire pulse light source 412 to be located at the side of the corresponding support member 22 facing towards the other support member 22. In this way, the irradiation area of the pulse unit 41 on the indoor air duct 100a is effectively ensured to better sterilize the indoor air duct 100a.

It should be understood that a position of the abutment portion 41111 is not limited in this regard. The position of the abutment portion 41111 in the length direction of the pulse unit 41 can be set as desired. For example, the position of the abutment portion 41111 in an axial direction of the mounting bracket 411 is adjusted to control a length of a part of the pulse unit 41 that extends out of the mounting hole 221 towards the other support member 22, and thus the irradiation area of the pulse unit 41 on the indoor air duct 100a is adjusted to adapt to a need for sterilization of the air conditioner 100.

The mounting bracket 411 is further provided with a fixing portion 41121 at the end of the mounting bracket 411 in the length direction of the mounting bracket 411. The fixing portion 41121 is located at a side of the corresponding support member 22 facing away from the other support member 22, and is fixed to the corresponding support member 22 by a fastener. In this case, a circumferential wall of the mounting hole 221 can provide support for the pulse unit 41, and the fixing portion 41121 can restrict the pulse unit 41 from detaching from the mounting hole 221, to ensure stable and reliable mounting of the sterilization module 4. In addition, the abutment portion 4111 can pre-restrict a position of the pulse unit 41, facilitating a quick connection of the fixing portion 41121 to the support member 22.

In some embodiments of the present disclosure, as illustrated in FIG. 8, the mounting bracket 411 comprises a first bracket 4111 and a second bracket 4112. The first bracket 4111 defines an assembly space. The assembly space is used to mount the pulse light source 412 to protect the pulse light source 412. The first bracket 4111 has the abutment portion 41111 formed at an end of the first bracket 4111 in a length direction of the first bracket 4111. The abutment portion 41111 is abutted against a side of the mounting hole 221 facing away from the other support member 22. The second bracket 4112 wraps the end of the first bracket 4111 in the length direction of the first bracket 4111 and comprises the fixing portion 41121. The fixing portion 41121 is located at the side of the corresponding support member 22 facing away from the other support member 22, and is fixed to the corresponding support member 22 by a fastener. With the first bracket 4111 and the second bracket 4112, mounting and fixation of the pulse unit 41 are facilitated.

As illustrated in FIG. 9 and FIG. 10, the first bracket 4111 comprises a protrusion and the second bracket 4112 comprises a snap. Through an engagement between the protrusion and the snap, fixation between the first bracket 4111 and the second bracket 4112 is realized, which ensures overall structural stability of the first bracket 4111 and the second bracket 4112. Further, a design of the protrusion and the snap facilitates a disassembly of the first bracket 4111 for replacement of the pulse light source 412, which eliminates a need to replace the entire sterilization module 4 due to damages to the pulse light source 412, saving later maintenance costs for the user.

In some embodiments of the present disclosure, the mounting bracket 411 is integrally formed.

In some embodiments of the present disclosure, as illustrated in FIG. 9 and FIG. 10, the mounting bracket 411 has a mounting space 411a formed therein. The mounting space 411a has an emission opening 411b. The pulse light source 412 is disposed in the mounting space 411a. In addition, the pulse light source 412 is arranged in correspondence with the emission opening 411b, in such a manner that at least a large portion of the pulsed intense light outputted by the pulse light source 412 can be centrally emitted through the emission opening 411b to at least sterilize the airflow, which facilitates further improving the sterilization efficiency and the sterilization effect. In addition, the mounting bracket 411 can protect the pulse light source 412 from friction of other members, ensuring a normal operation of the pulse light source 412.

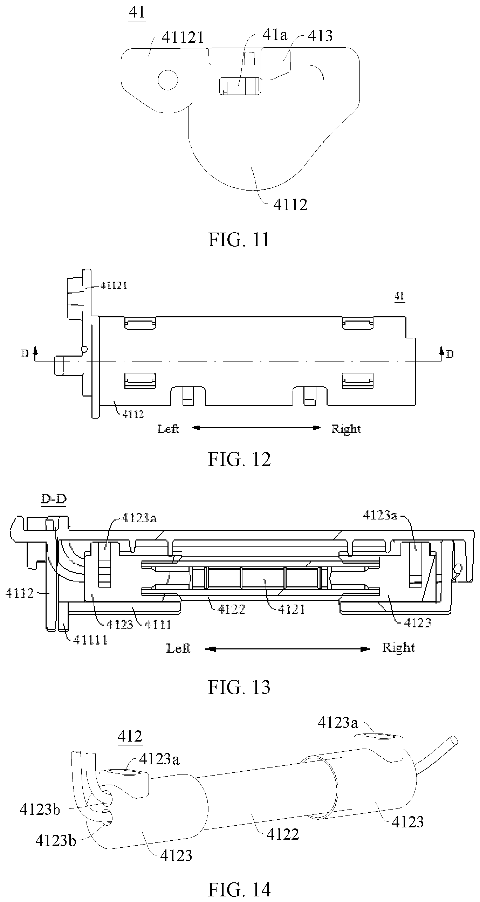

For example, as illustrated in FIG. 13, the mounting bracket 411 and the pulse light source 412 both extend in the left-right direction. The emission opening 411b is formed at a bottom side of the mounting space 411a. The pulse light source 412 is located below the mounting bracket 411. At least part of the pulse light source 412 is disposed in the mounting space 411a. When the sterilization module 4 is applied in the air conditioner 100, the emission opening 411b may be arranged towards the indoor air duct 100a and an internal member (e.g., the indoor fan 3), in such a manner that the sterilization module 4 can simultaneously sterilize the indoor air duct 100a and the internal member. Other members of the air conditioner 100 can also be sterilized by the sterilization module 4 when these members are within the irradiation coverage of the pulse light source 412.

In some embodiments of the present disclosure, as illustrated in FIG. 13, the pulse light source 412 comprises the xenon lamp 4121, a lampshade 4122, and a seal 4123. The xenon lamp 4121 is filled with inert gases comprising the xenon gas, which causes no pollution and is not hazardous to the human body. The lampshade 4122 is sleeved over the xenon lamp 4121 and has openings formed at two axial ends of the lampshade 4122, respectively, in such a manner that the xenon lamp 4121 is engaged in the lampshade 4122 through the openings. The seal 4123 is sealed at the opening. The seal 4123 and the lampshade 4122 defines a mounting cavity configured to accommodate the xenon lamp 4121. In this way, the two seals 4123 can seal the xenon lamp 4121 in the mounting cavity, which is conducive to reducing a danger during mounting and a disassembly of the pulse light source 412. The lampshade 4122 is a transparent member, which improves light transmittance of the pulsed intense light released by the xenon lamp 4121, and reduces an energy loss of the pulse light source 412.

In some embodiments of the present disclosure, the lampshade 4122 is a glass member (e.g., a quartz member), a crystal member, etc., to ensure high light transmittance of the lampshade 4122, which facilitates ensuring the sterilization effect of the pulse light source 412.

In some embodiments of the present disclosure, the seal 4123 is a silicone member or a rubber member, to facilitate provision of a soft connection while ensuring sealing performance of the mounting cavity. A part of the seal 4123 is sleeved over an end of the lampshade 4122 to provide protection for the lampshade 4122 by the seal 4123. In this way, the lampshade 4122 is prevented from coming into direct contact with other members to avoid cracks or the like in the lampshade 4122, which is conducive to improving operational safety and prolonging a service life of the pulse light source 412.

For example, as illustrated in FIG. 14, the seal 4123 has an engagement groove at a side of the seal 4123 facing towards the lampshade 4122. The engagement groove is formed as a ring-shaped groove. The lampshade 4122 is inserted into and engaged with the engagement groove. An insulating sealant is disposed between a wall surface of the ring-shaped groove and the lampshade 4122 to provide sealing.

The mounting bracket 411 further comprises the first bracket 4111. Each of the two seals 4123 is mounted at the first bracket 4111 to realize mounting of the xenon lamp 4121, which facilitates stable mounting of the pulse light source 412 as a whole and prevents a vibration generated by an operation of the air conditioner 100 from changing a position of the xenon lamp 4121, improving stability of the xenon lamp 4121.

In some embodiments, as illustrated in FIG. 13 and FIG. 14, at least one of the two seals 4123 has a chamber in communication with the mounting cavity. The chamber has a through opening 4123a and a cable opening 4123b. A cable of the xenon lamp 4121 (e.g., a positive cable, a negative cable, and a breakdown cable of the xenon lamp 4121) extends out of the chamber through the through opening 4123a or the cable opening 4123b. The chamber can provide a predetermined route for an exit of the cable of the xenon lamp 4121. In addition, the seal 4123 and the first bracket 4111 can provide insulation protection for the cable of the xenon lamp 4121 to avoid safety hazards during use, and to avoid a need for a heat shrink tubing application of the cable of the xenon lamp 4121 in a small space, which facilitates improving mounting safety of the pulse light source 412.

For example, in examples of FIG. 13 and FIG. 14, each of the two seals 4123 has the chamber. Each chamber has the cable opening 4123b. One terminal of the xenon lamp 4121 is a positive terminal, while another terminal of the xenon lamp 4121 is a negative terminal. Each of the positive terminal and the negative terminal is provided with the cable. The cable at the positive terminal can be inserted into the chamber of one of the two seals 4123 and extend out of the chamber through the cable opening 4123b of the one of the two seals 4123. The cable at the negative terminal can be inserted into the chamber of another one of the two seals 4123 and extend out of the chamber through the cable opening 4123b of the other one of the two seals 4123. Alternatively, the cable at the positive terminal and the cable at the negative terminal both extend into the chamber of a same seal 4123 and extend out of the chamber through the cable opening 4123b of the same seal 4123.

The xenon lamp 4121 is further provided with the breakdown cable at the positive terminal or the negative terminal of the xenon lamp 4121. The breakdown cable may extend into the chamber of one of the two seals 4123 and extend out of the chamber through the cable opening 4123b of the one of the two seals 4123.

The chamber is provided with the sealant to further ensure sealing performance of the mounting cavity, avoiding an intrusion of other foreign matters (e.g., water vapors) into the mounting cavity. For example, the sealant such as silicone rubber is injected into the chamber through the through opening 4123a. In this case, the through opening 4123a may be formed at a side wall of the seal 4123 that extends in a circumferential direction of the xenon lamp 4121. After the seal 4123 is mounted at the lampshade 4122, if the through opening 4123a is not oriented upwards, the seal 4123 may be rotated to orient the through opening 4123a upwards, in such a manner that the sealant is prevented from flowing out during an injection of the sealant, which facilitates an operation and assurance of cleanliness of a workbench and the lampshade 4122. The insulating sealant is disposed at the through opening 4123a.

In some embodiments of the present disclosure, as illustrated in FIG. 8 and FIG. 13, the abutment portion 41111 extends into a ring-shaped structure in a circumferential direction of the mounting hole 221, and is sealingly engaged with an edge of the mounting hole 221. In this way, the abutment portion 41111 can be sealingly engaged with an entire circumferential edge of the mounting hole 221 to prevent the airflow in the indoor air duct 100a from flowing out through a gap between the mounting bracket 411 and the mounting hole 221, which avoids an air leakage of the air conditioner 100, facilitating assurance of an air discharge volume of the air conditioner 100. In addition, the foreign matters (such as dust) can be prevented from entering the indoor air duct 100a through the gap between the mounting bracket 411 and the mounting hole 221, which prevents a normal operation of the members in the air conditioner 100 from being affected.

In some embodiments of the present disclosure, as illustrated in FIG. 8, at least one of two opposite sides of the corresponding support member 22 in an axial direction of the mounting hole 221 has a limiting rib 222. The limiting rib 222 is arranged around the mounting hole 221 and engaged with an outer peripheral side of the pulse unit 41, to restrict a movement of the pulse unit 41 in a direction perpendicular to the length direction of the heat exchanger body 21 to a predetermined extent, ensuring that the mounting bracket 411 is stably engaged with the mounting hole 221.

For example, in the example of FIG. 8, the corresponding support member 22 at which the sterilization module 4 is mounted has the limiting rib 222 at a side of the corresponding support member 22 facing towards the other support member 22. The limiting rib 222 extends along the edge of the mounting hole 221, and extends from the edge of the mounting hole 221 towards the other support member 22. In this way, the limiting rib 222 can also provide support for the pulse unit 41. The corresponding support member 22 at which the sterilization module 4 is mounted has the limiting rib 222 at the side of the corresponding support member 22 facing away from the other support member 22. The limiting rib 222 is disposed at a radially outer side of the edge of the mounting hole 221. The limiting rib 222 defines a recess 223. A part of the pulse unit 41 located at the side of the corresponding support member 22 facing away from the other support member 22 is engaged with the recess 223. The recess 223 is used to confine the pulse unit 41 to provide a positional restriction and support, which is conducive to positioning and the mounting of the pulse unit 41.

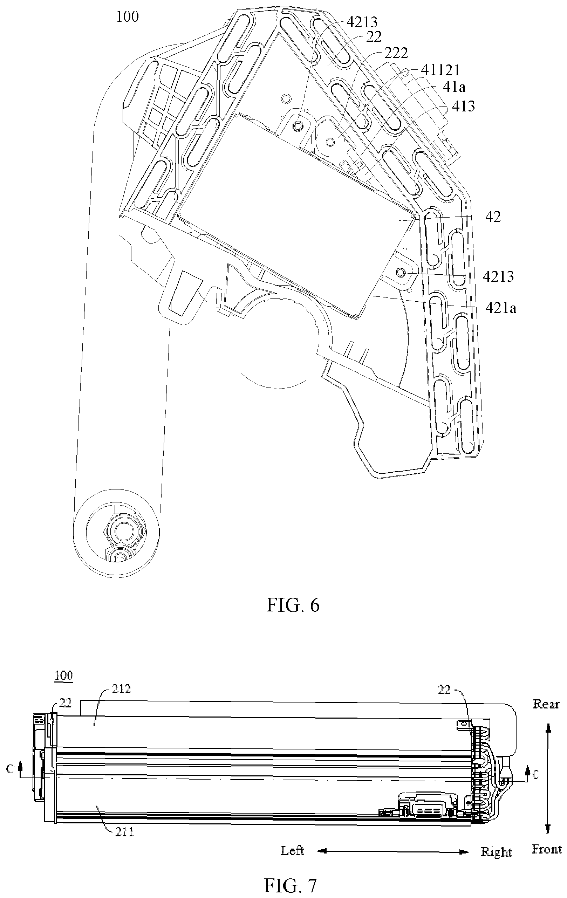

In some embodiments of the present disclosure, as illustrated in FIG. 6 and FIG. 8, the sterilization module 4 further comprises a circuit unit 42. The circuit unit 42 comprises a box 421 and a circuit board 422. The box 421 is disposed at the side of the corresponding support member 22 (the corresponding support member 22 at which the sterilization module 4 is mounted) facing away from the other support member 22, and is fixed to the corresponding support member 22 by a fastener. The circuit board 422 is disposed in the box 421, and is electrically connected to the pulse unit 41, which facilitates provision of a suitable operation voltage for the pulse unit 41, and control over activation, deactivation, and an operational duration (e.g., the operational duration may range from two minutes to five minutes) of the pulse unit 41, conveniently adapting to a need of the user. In addition, the circuit unit 42 can be arranged without occupying a space between the two support members 22, freeing up more space for the mounting of the pulse unit 41.

In this case, at least part of the pulse unit 41 and the circuit board 42 are located at two opposite sides of the support member 22, respectively. If the support member 22 has the mounting hole 221, the cable between the pulse unit 41 and the circuit board 42 may be routed through the mounting hole 221. If the support member 22 has no mounting hole 221, the cable between the pulse unit 41 and the circuit board 42 may be routed bypassing the support member 22, but is not limited to being routed in such a manner.

The sterilization module 4 is constructed to perform sterilization once every 24 hours, and the duration of each sterilization is 2 min to 5 min.

In some embodiments, as illustrated in FIG. 5 and FIG. 6, the indoor heat exchanger 2 comprises a heat exchanger body 21. The heat exchanger body 21 comprises a first heat exchange portion 211 and a second heat exchange portion 212. The first heat exchange portion 211 is angled relative to the second heat exchange portion 212. The box 421 has a square structure. A part of the box is accommodated in a space, corresponding to a space defined by the angle between the first heat exchange portion 211 and the second heat exchange portion 212, at the side of the corresponding support member 22 facing away from the other support member 22. A length direction of the square structure is arranged obliquely relative to a vertical direction. A width direction of the square structure is the same as an oblique direction of the second heat exchange portion 212. In this way, a sufficient mounting space can be provided for the box 421, and the box 421 does not interfere with a mounting portion of the indoor fan 3.

In some embodiments of the present disclosure, the box 421 is filled with a sealant wrapping around the circuit board 422 to prevent water vapors and other foreign matters from causing short circuits, damages, or the like to the circuit board 422, which ensures a safe operation of the circuit board 422, and enhances reliability of electric control of the circuit unit 42. The sealant can wrap all of or a part of the circuit board 422.

The sealant is a polyurethane adhesive. For example, the circuit board 422 (e.g., a PCB circuit board) is sealed with the polyurethane adhesive. Also, the sealant provides 100% coverage of all pins of the circuit board 422 to avoid damages to the circuit board 422.

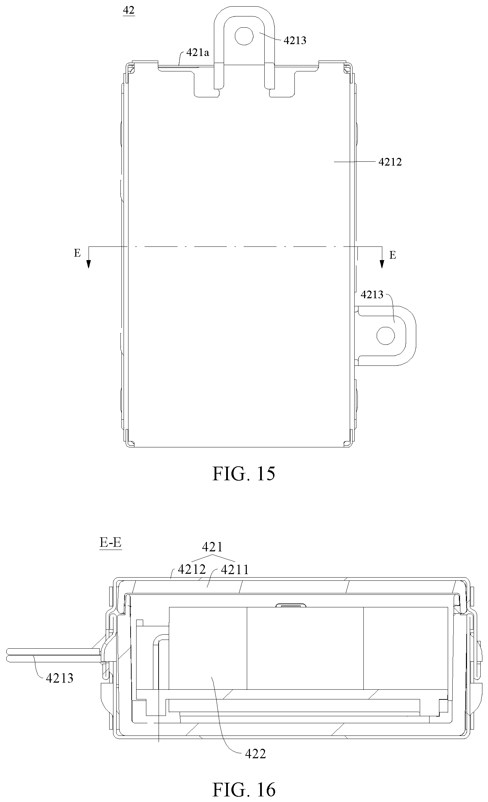

In some embodiments of the present disclosure, as illustrated in FIG. 16, the box 421 comprises a first housing 4211 and a second housing 4212. The circuit board 422 is disposed in the first housing 4211. The second housing 4212 wraps around the first housing 4211. In this way, the second housing 4212 can provide protection for the first housing 4211 and the circuit board 422. With the first housing 4211 and the second housing 4212, dual-layer protection can be provided for the circuit board 422, ensuring safety of the circuit board 422.

The first housing 4211 is a plastic member. The second housing 4212 is a sheet metal member. The first housing 4211 can provide satisfactory moisture and water resistance to effectively protect the circuit board 422 from being affected by condensed water at the support member 22 (e.g., condensed water generated by a support plate adjacent to the indoor heat exchanger 2 due to an operation of the indoor heat exchanger 2). The second housing 4212 has excellent fireproof and explosion-proof capabilities, thereby providing the circuit unit 42 with good waterproof and explosion-proof performance, further enhancing use safety of the circuit unit 42.

In some embodiments of the present disclosure, as illustrated in FIG. 6, FIG. 8, and FIG. 11, the pulse unit 41 has a first lead opening 41a at an end of the pulse unit 41 in a length direction of the pulse unit 41. The box 421 has a second lead opening 421a at a lower side of the box 421. A cable of the pulse unit 41 is connected to the circuit board 422 through the first lead opening 41a and the second lead opening 421a. In this way, an arrangement of the cable of the pulse unit 41 is facilitated. In addition, in a case where the indoor heat exchanger 2 is used as an evaporator, if condensed water is generated at a surface of the box 421, a surface of the support member 22, etc., the condensed water will not flow into the box 421 through the second lead opening 421a since the second lead opening 421a is located at the lower side of the box 421, ensuring a normal operation of the circuit unit 42. Further, in view of a position of the second lead opening 421a, a part of the cable of the pulse unit 41 passes through the second lead opening 421a from bottom to up when the cable of the pulse unit 41 is connected to the circuit board 422 through the second lead opening 421a. In this way, even if condensed water presents at a surface of the cable, the condensed water will not flow along the cable towards the second lead opening 421a, which further enhances water resistance of the sterilization module 4.

The sterilization module 4 is mounted at the support member 22 at the left side. A main electric control box of the air conditioner 100 is mounted at a right side of the indoor heat exchanger 2. A wire of the circuit board 422 may extend out of the second lead opening 421a, and is connected to a main electric control board of the air conditioner 100 under guidance of a plurality of wire clips at a base of the air conditioner 100, in such a manner such that the cable of the pulse unit 41 is electrically connected to the main electric control board by the circuit board 422.

In some embodiments of the present disclosure, as illustrated in FIG. 6 and FIG. 9, the pulse unit 41 further has a cable retention structure 413 at an end of the pulse unit 41 in the length direction of the pulse unit 41. The cable retention structure 413 is located between the first lead opening 41a and the second lead opening 421a. The cable of the pulse unit 41 passes through the cable retention structure 413. In this way, a centralized arrangement of a part of the cable located between the first lead opening 41a and the second lead opening 421a is facilitated, which restricts a movement of the cable to prevent the cable from affecting an operation of other members.

In some embodiments of the present disclosure, a part of the cable of the pulse unit 41 is sandwiched between the box 421 and the corresponding support member 22. That is, the part of the cable of the pulse unit 41 located between the first lead opening 41a and the second lead opening 421a is sandwiched between the box 421 and the corresponding support member 22, which restricts the movement of the cable to prevent the cable from interfering with other members.

The part of the cable of the pulse unit 41 located between the first lead opening 41a and the second lead opening 421a may also be arranged around an outer peripheral side of the box 421.

For example, in an example of FIG. 6, the pulse unit 41 further has the cable retention structure 413 at the end of the pulse unit 41 in the length direction of the pulse unit 41. The cable retention structure 413 is located between the first lead opening 41a and the second lead opening 421a. The cable of the pulse unit 41 extends through the first lead opening 41a to the side of the corresponding support member 22 facing away from the other support member 22, passes through the cable retention structure 413, and then passes downwards through a space between the box 421 and the corresponding support member 22 to be sandwiched between the box 421 and the corresponding support member 22. Then, the cable of the pulse unit 41 is arranged around a mounting post 4213 at the lower side of the box 421, and is connected to the circuit board 422 through the second lead opening 421a. The mounting post 4213 is fixed to the support member 22 by a fastener to realize mounting and fixation of the circuit unit 22.

In some embodiments of the present disclosure, as illustrated in FIG. 2, the sterilization module 4 is located between the indoor heat exchanger 2 and the indoor fan 3. The sterilization module 4 is configured to irradiate the indoor heat exchanger 2 and the indoor fan 3 to sterilize at least one of the indoor heat exchanger 2 and the indoor fan 3. In this way, the indoor heat exchanger 2 or the indoor fan 3 is sterilized by the pulse light source 412 to adapt to a need for the air conditioner 100 and ensure cleanliness of the indoor heat exchanger 2 or the indoor fan 3.

It can be appreciated that when the sterilization module 4 sterilizes the indoor heat exchanger 2, the pulse unit 41 may irradiate a part or all of the indoor heat exchanger 2 to achieve sterilization. That is, when the pulse unit 41 is in operation, at least part of the indoor heat exchanger 2 is within the irradiation coverage of the pulsed intense light outputted by the pulse unit 41. Therefore, a relative position between the pulse light source 412 and the indoor heat exchanger 2 can be flexibly set to some extent. Similarly, when the sterilization module 4 sterilizes the indoor fan 3, the pulse unit 41 may irradiate a part or all of the indoor fan 3 to achieve sterilization. That is, when the pulse unit 41 is in operation, at least part of the indoor fan 3 is within the irradiation coverage of the pulsed intense light outputted by the pulse unit 41.

Therefore, an irradiation direction of the pulsed intense light of the sterilization module 4 can be adapted to a mounting position of the sterilization module 4 to ensure a sterilization range of the pulsed intense light.

In some embodiments of the present disclosure, as illustrated in FIG. 2 to FIG. 5, the indoor heat exchanger 2 comprises the heat exchanger body 21. The heat exchanger body 21 comprises the first heat exchange portion 211 and the second heat exchange portion 212. The first heat exchange portion 211 is angled relative to the second heat exchange portion 212. An end of the first heat exchange portion 211 and an end of the second heat exchange portion 212 that are close to each other face towards an air intake side of the air conditioner 100. The sterilization module 4 is disposed between the first heat exchange portion 211 and the second heat exchange portion 212, and is located at an air intake side of the indoor fan 3. In this way, mounting of the sterilization module 4 in the space defined by the angle between the first heat exchange portion 211 and the second heat exchange portion 212 is facilitated, in such a manner that a sufficient arrangement space is provided for the sterilization module 4. Therefore, a structural arrangement of the heat exchanger body 21 and the sterilization module 4 is made more compact, which facilitates reasonable utilization of an internal space of the air conditioner 100. Also, a sterilization area of the sterilization module 4 on the airflow, the heat exchanger body 21, and the indoor fan 3 is ensured, which is conducive to realizing all-around sterilization of the indoor air duct 100a by the sterilization module 4.

For example, in an example of FIG. 2, the sterilization module 4 is disposed at an upper side of the indoor fan 3 (e.g., the sterilization module 4 is located at a directly upper side or an obliquely upper side of the indoor fan 3). The pulsed intense light of the sterilization module 4 is arranged to irradiate the indoor air duct 100a obliquely relative to the vertical direction, but is not limited to being arranged in such a manner.

In some embodiments of the present disclosure, as illustrated in FIG. 2, a minimum spacing between the sterilization module 4 and the indoor heat exchanger 2 is x1, where x1≥8 mm; and/or a minimum spacing between the sterilization module 4 and the indoor fan 3 is x2, where x2≥15mm. Therefore, the sterilization module 4 is at a predetermined safety distance from other members while ensuring reliable mounting of the sterilization module 4, which avoids interference between the sterilization module 4 and other members (e.g., the indoor heat exchanger 2 and the indoor fan 3), preventing the normal operation of the sterilization module 4 and other members from being affected.

x1 may be 8 mm, 10 mm, 12 mm, etc., while x2 may be 15 mm, 18 mm, 25 mm, etc. In an example of the figure, a minimum spacing between a lowest end of the sterilization module 4 and the indoor fan 3 is greater than or equal to 15 mm.

It should be noted that, in the description of the present disclosure, "and/or" is meant to comprise three parallel solutions. For example, "A and/or B" comprises solution A, solution B, or both solution A and solution B.

In some embodiments of the present disclosure, as illustrated in FIG. 2 to FIG. 5, the air conditioner 100 further comprises an electric auxiliary heating module 5. The electric auxiliary heating module 5 is located between the indoor heat exchanger 2 and the indoor fan 3. Two ends of the electric auxiliary heating module 5 in a length direction of the electric auxiliary heating module 5 are fixedly connected to the two support members 22 of the indoor heat exchanger 2, respectively, which facilitates mounting of the electric auxiliary heating module 5 to improve stability of the electric auxiliary heating module 5, and is conducive to increasing a heat exchange area between the electric auxiliary heating module 5 and the airflow in the indoor air duct 100a.

The electric auxiliary heating module 5 and the sterilization module 4 are offset from each other in a direction perpendicular to the length direction of the electric auxiliary heating module 5, which facilitates a reasonable layout of the electric auxiliary heating module 5 and the sterilization module 4, and is conducive to taking into account both an electric auxiliary heating effect and a sterilization effect of the air conditioner 100.

As an example, the length direction of the electric auxiliary heating module 5 is the left-right direction. The electric auxiliary heating module 5 and the sterilization module 4 are offset from each other in an up-down direction and/or a front-rear direction. In this way, in a plane perpendicular to the length direction of the electric auxiliary heating module 5, an orthographic projection of the electric auxiliary heating module 5 and an orthographic projection of the sterilization module 4 are at least partially non-overlapping. For example, the orthographic projection of the electric auxiliary heating module 5 may be spaced apart from the orthographic projection of the sterilization module 4.

For example, in examples of FIG. 2 and FIG. 5, the sterilization module 4 is disposed at a lower side of the electric auxiliary heating module 5 (e.g., the sterilization module 4 is located at a directly lower side or an obliquely lower side of the electric auxiliary heating module 5). The pulsed intense light of the sterilization module 4 irradiates downwards at the indoor air duct 100a. The sterilization module 4 may also be disposed at an upper side of the electric auxiliary heating module 5 (e.g., the sterilization module 4 is located at a directly upper side or an obliquely upper side of the electric auxiliary heating module 5), but is not limited to being disposed in such a manner.

In some embodiments of the present disclosure, as illustrated in FIG. 2, the sterilization module 4 is located at the obliquely lower side of the electric auxiliary heating module 5, in such a manner that the sterilization module 4 and the electric auxiliary heating module 5 may be offset from each other in the front-rear direction. The emission opening 411b of the sterilization module 4 is arranged obliquely downwards, which is convenient for enabling the irradiation coverage of the sterilization module 4 to be located below the electric auxiliary heating module 5, helping to maximize the irradiation coverage of the pulse unit 41. In addition, an outer contour of the sterilization module 4 can be better adapted to an arrangement space at the lower side of the electric auxiliary heating module 5, which is conducive to keeping the sterilization module 4 at a safe distance from other members such as the indoor heat exchanger 2 and the indoor fan 3. In addition, it is convenient to offset the irradiation coverage of the sterilization module 4 from the electric auxiliary heating module 5 to further ensure the irradiation coverage of the pulse unit 41, preventing the electric auxiliary heating module 5 from blocking the emission opening 411b of the sterilization module 4.

In some embodiments of the present disclosure, the length direction of the electric auxiliary heating module 5 is the same as the length direction of the pulse unit 41. Such an arrangement facilitating both a heating area of the electric auxiliary heating module 5 on the airflow in the indoor air duct 100a and an irradiation area of the pulse unit 41 on the airflow in the indoor air duct 100a, which is conducive to taking into account both the electric auxiliary heating effect of the electric auxiliary heating module 5 and the sterilization effect of the sterilization module 4, and helps to reduce a space occupied by the sterilization module 4 in the plane perpendicular to the length direction of the electric auxiliary heating module 5, further facilitating the arrangement of the sterilization module 4 in the air conditioner 100.

For example, a center axis of the electric auxiliary heating module 101 in the length direction of the electric auxiliary heating module 101 is parallel to and spaced apart from a center axis of the sterilization module 3 in a length direction of the sterilization module 3.

In some embodiments, as illustrated in FIG. 1, the casing 1 has a display panel. The display panel is configured to display at least one of parameter information, mode information, and the like related to the air conditioner 100. For example, the display panel may display information related to temperature (e.g., a set temperature of the air conditioner 100), air sensation, humidity, purification, fresh air, and sterilization.

In some embodiments of the present disclosure, surrounding members (e.g., the support member 22 or the electric auxiliary heating module 5) of the sterilization module 4 are all made of weather-resistant, heat-resistant, and anti-aging materials to withstand ultraviolet rays from the pulsed intense light. In addition, the sterilization module 4 is mounted at a periphery of the electric auxiliary heating module 5. The cable of the pulse unit 41 is a heat-resistant and anti-aging cable to ensure a service life of the air conditioner 100. If cables of other members are also in the irradiation coverage of the pulsed intense light, the cables of other members may also be heat-resistant and anti-aging cables.

In the description of the present disclosure, it should be understood that, the orientation or the position indicated by technical terms such as "center", "longitudinal", "transverse", "length", "width", "thickness", "over", "below", "front", "rear", "left" , "right", "vertical", "horizontal", "top", "bottom", "inner", "outer", "clockwise", "anti-clockwise", "axial", "radial", and "circumferential" should be construed to refer to the orientation or the position as shown in the drawings, and is only for the convenience of describing the embodiments of the present disclosure and simplifying the description, rather than indicating or implying that the pointed device or element must have a specific orientation, or be constructed and operated in a specific orientation, and therefore cannot be understood as a limitation of the present disclosure.

In addition, terms "first" and "second" are only used for descriptive purposes, and cannot be understood as indicating or implying relative importance or implicitly indicating the number of indicated technical features. Therefore, the features associated with "first" and "second" may explicitly or implicitly comprise at least one of the features. In the description of the present disclosure, "plurality" means at least two, unless otherwise specifically defined.

In the present disclosure, unless otherwise clearly specified and limited, terms such as "install", "connect", "connect to", "fix" and the like should be understood in a broad sense. For example, it may be a fixed connection or a detachable connection or connection as one piece; mechanical connection or electrical connection; direct connection or indirect connection through an intermediate; internal communication of two components or the interaction relationship between two components, unless otherwise clearly limited. For those skilled in the art, the specific meaning of the above-mentioned terms in the present disclosure can be understood according to specific circumstances.

In the present disclosure, unless expressly stipulated and defined otherwise, the first feature "on" or "under" the second feature may mean that the first feature is in direct contact with the second feature, or the first and second features are in indirect contact through an intermediate. Moreover, the first feature "above" the second feature may mean that the first feature is directly above or obliquely above the second feature, or simply mean that the level of the first feature is higher than that of the second feature. The first feature "below" the second feature may mean that the first feature is directly below or obliquely below the second feature, or simply mean that the level of the first feature is smaller than that of the second feature.

Reference throughout this specification to terms such as "an embodiment", "some embodiments", "an example", "a specific example", or "some examples" means that a particular feature, structure, material, or characteristic described in connection with the embodiment or example is comprised in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples. In addition, different embodiments or examples and features of different embodiments or examples described in the specification may be combined by those skilled in the art without mutual contradiction.

Although embodiments of the present disclosure have been shown and described above, it should be understood that above embodiments are merely exemplary, and cannot be construed to limit the present disclosure. For those skilled in the art, changes, alternatives, and modifications can be made to the embodiments without departing from the scope of the present disclosure.

## Claims

1. An air conditioner, comprising:
a casing defining an indoor air duct therein;
an indoor heat exchanger disposed in the indoor air duct;
an indoor fan disposed in the indoor air duct; and
a sterilization module disposed in the casing, the sterilization module comprising a pulse unit configured to output pulsed intense light to at least sterilize airflow in the indoor air duct.

2. The air conditioner according to claim 1, wherein the indoor heat exchanger comprises:
a heat exchanger body; and
two support members respectively disposed at two ends of the heat exchanger body in a length direction of the heat exchanger body, at least one of the two support members being provided with the sterilization module, and at least a part of the pulse unit being located at a side of a corresponding support member facing towards an other support member.

3. The air conditioner according to claim 2, wherein the support member is formed with a mounting hole, the pulse unit passing through the mounting hole and being fixedly connected to the support member.

4. The air conditioner according to claim 3, wherein the pulse unit comprises a mounting bracket and a pulse light source disposed at the mounting bracket, the mounting bracket being provided with an abutment portion and a fixing portion at an end of the mounting bracket in a length direction of the mounting bracket, wherein:
the abutment portion is abutted against a side of the mounting hole facing away from the other support member; and
the fixing portion is located at a side of the support member facing away from the other support member, and is fixed to the support member by a fastener.

5. The air conditioner according to claim 4, wherein the abutment portion extends into a ring-shaped structure in a circumferential direction of the mounting hole, and is sealingly engaged with an edge of the mounting hole.

6. The air conditioner according to claim 4 or 5, wherein at least one of two opposite sides of the support member in an axial direction of the mounting hole has a limiting rib, the limiting rib being arranged around the mounting hole and engaged with an outer peripheral side of the pulse unit.

7. The air conditioner according to any one of claims 2 to 6, wherein the sterilization module further comprises a circuit unit, the circuit unit comprising a box and a circuit board, wherein:
the box is disposed at the side of the corresponding support member facing away from the other support member, and is fixed to the corresponding support member by the fastener; and
the circuit board is disposed in the box, and is electrically connected to the pulse unit.

8. The air conditioner according to claim 7, wherein the box is filled with a sealant, the sealant wrapping the circuit board.

9. The air conditioner according to claim 7 or 8, wherein the box comprises:
a first housing, the circuit board being disposed in the first housing, and the first housing being a plastic member; and
a second housing wrapping around the first housing, the second housing being a sheet metal member.

10. The air conditioner according to any one of claims 7 to 9, wherein:
the pulse unit has a first lead opening at an end of the pulse unit in a length direction of the pulse unit; and
the box has a second lead opening formed at a lower side of the box, a cable of the pulse unit being connected to the circuit board through the first lead opening and the second lead opening.

11. The air conditioner according to claim 10, wherein the pulse unit further has a cable retention structure at the end of the pulse unit in the length direction of the pulse unit, the cable retention structure being located between the first lead opening and the second lead opening, and the cable of the pulse unit passing through the cable retention structure.

12. The air conditioner according to claim 10 or 11, wherein a part of the cable of the pulse unit is sandwiched between the box and the corresponding support member.

13. The air conditioner according to any one of claims 1 to 12, wherein the sterilization module is located between the indoor heat exchanger and the indoor fan, and is configured to irradiate the indoor heat exchanger and the indoor fan to sterilize at least one of the indoor heat exchanger and the indoor fan.

14. The air conditioner according to claim 13, wherein the indoor heat exchanger comprises a heat exchanger body, the heat exchanger body comprising a first heat exchange portion and a second heat exchange portion, wherein:
the first heat exchange portion is angled relative to the second heat exchange portion;
an end of the first heat exchange portion and an end of the second heat exchange portion, which are close to each other, are arranged to face towards an air intake side of the air conditioner; and
the sterilization module is disposed between the first heat exchange portion and the second heat exchange portion, and is located at an air intake side of the indoor fan.

15. The air conditioner according to claim 13 or 14, wherein:
a minimum spacing between the sterilization module and the indoor heat exchanger is x1, where x1≥8 mm; and/or
a minimum spacing between the sterilization module and the indoor fan is x2, where x2≥15 mm.

16. The air conditioner according to any one of claims 13 to 15, further comprising an electric auxiliary heating module, wherein:
the electric auxiliary heating module is located between the indoor heat exchanger and the indoor fan;
two ends of the electric auxiliary heating module in a length direction of the electric auxiliary heating module are fixedly connected to the two support members of the indoor heat exchanger, respectively; and
the electric auxiliary heating module and the sterilization module are offset from each other in a direction perpendicular to the length direction of the electric auxiliary heating module.

17. The air conditioner according to claim 16, wherein:
the sterilization module is located at an obliquely lower side of the electric auxiliary heating module; and
the sterilization module has an emission opening arranged obliquely downwards.
